# EUROPEAN PATENT APPLICATION

(11) **EP 2 839 855 A2**
(43) Date of publication of application: **25.02.2015**
(21) Application number: 14181171.1
(22) Date of filing: 19.10.2010
(51) Int. Cl.: A61M 5/32, A61M 5/315, A61M 5/34

(54) **Syringe assemblies having detachable needle assemblies and low dead space**

(30) Priority: 19.10.2009 US 252962 P
(62) Divisional of application: 10188014.4
(71) Applicant: Terumo Medical Corporation, Somerset, New Jersey 08873 (US)
(72) Inventor: Matthews, David, Joppa, MA Massachusetts 21085 (US); Voellmicke, Craig, New York, NY New York 10023 (US); Powers, Thomas, Newtown, PA Pennsylvania 18940 (US); Dowdell, Ralph, Trenton, NJ New Jersey 08611 (US); Hidalgo, Craig, Langhorne, PA Pennsylvania 19047 (US); Bredael, Gary, Langhorne, PA Pennsylvania 19053 (US)
(74) Representative: Jenkins, Peter David

(57) **Abstract**

The present invention is directed to syringe assemblies comprising detachable needle assemblies, that are configured in such a manner as to minimize the dead space after deployment of the syringe piston and injection of the material in the syringe into a patient's body, as well as with bevel indicators that provide guidance as to optimal angles at which to inject the needles into patients.

## Description

### CROSS REFERENCE TO RELATED APPLICATIONS

This application claims benefit of U.S. Provisional Application No. 61/252,962 filed October 19, 2009, hereby incorporated by reference in its entirety.

### BACKGROUND OF THE INVENTION

The present invention is related to the field of hypodermic syringes and needles, and particularly to syringe assemblies comprising detachable needle assemblies.

Hypodermic syringes are commonly used by doctors, nurses and other medical staff (including staff trained in cosmetic procedures) for various medical and cosmetic procedures that require injection of medication and other medical or cosmetic substances into patient's bodies. Such syringes are also commonly used by certain patients, as for example, diabetics who may self-inject insulin.

In certain situations where a patient must be given multiple injections, for example for allergy injections, injections of insulin for diabetics or cosmetic applications, it is desirable to avoid dulling of a hypodermic needle over multiple uses, as dulling can lead to decreased accuracy in drug delivery discomfort for the patient. Further, in view of recent industry-wide attention to minimizing medical waste environmental impact, it is desirable to provide syringe assemblies that permit convenient use and interchangeability of needles with syringes, such as providing a single syringe that can be used multiple times on a single patient, where a used needle attached to the barrel of a syringe can quickly and easily be replaced with a fresh and sterile needle.

Even further, in view of the increased cost of healthcare, it is further desirable to provide syringe assemblies that are configured to maximize the fluid injected into a patient's body, thereby decreasing the "dead space" after discharge of the fluid, and thus diminishing uninjected fluid retained in the syringe after injection.

In general, an injection is administered in a manner including a "loading" step-that is, a step wherein the administrator or patient obtains a fully assembled but empty syringe assembly with the plunger fully depressed into the syringe barrel, then "loads" the syringe assembly by inserting the needle portion into a source of fluid (for example, a container that holds a reservoir of the fluid to be injected into the patient). This step is followed by the administrator or patient's pulling out the plunger by sliding it along the longitudinal axis of the barrel in a proximal direction, engaging a vacuum force to load the syringe with a predetermined amount of fluid. The needle is then withdrawn from the container, fully loaded and ready to inject into the patient. Repeated loading can also dull the needle.

Small needles have not traditionally been commonly used, but are gaining in popularity. For example, needles as small as 29-30 gauge are often used to inject insulin, and even needles as small as 31-33 gauge are now becoming more widely used (the "gauge" of a needle is a way of denoting the outer diameter of the needle, and a higher gauge indicates a small needle). Some uses for lower gauge needles include injecting insulin, administering allergy injections and for cosmetic applications such as the administration of Botox®. Further, allergy injections and Botox® injections generally require multiple injections to a patient in a single treatment. Thus, it may be desirable to use smaller needles for comfort and convenience for the patient. However, such needles are generally fragile because of their smaller size, and tend to be undesirable for loading, as their small circumference leads to slow loading and an increased risk of breakage during the handling of the syringe before injection. Moreover, multiple injections and the need to insert the needle through a barrier the top of a bottle or vial of sterile fluid as part of the loading step may all lead to faster dulling of the needle, causing further discomfort to the patient. Thus, the needles that are desirable for certain injections because of their small size are at the same time not so desirable for loading such syringes before injection into the patient. Yet, in the syringe and needle assemblies in the current art, users generally do not have the option of using one needle to load the syringe and another to inject the fluid into the patient. While a large needle may be fast to load, it may cause greater physical pain to the patient during injection, and such pain is multiplied with multiple injections. On the other hand, a small needle can be difficult to load. Thus, a user is often left with two undesirable alternatives.

In the situations where repeated loadings and injections on a single patient are desirable, there are other disadvantages besides increased possibility of breakage and/or dulling of the needle. Because many small syringe assemblies do not have detachable needles, a user may choose to use a fresh syringe for each injection to avoid dulling the needle. Discarding each syringe can be expensive and wasteful. Further, repeated injections over time may incrementally increase wasted fluid, as each injection will result in some fluid that is caught in the "dead space" in the syringe assembly rather than injected into the patient. This can be very expensive in the case of high-cost cosmetic fluid preparations such as Botox®.

Still further, many hypodermic needles are designed with a bevel tip that is cut at an angle, to facilitate smooth injection. The angle of such tip is known as the "bevel angle." Most bevel tips are cut as a "regular" bevel, which makes the tip appropriate for intramuscular delivery. However, other types of bevels include "short" bevels and "intradermal" bevels (all are based on the angle of the tip). To minimize a patient's discomfort, it is generally desirable to orient a needle such that when injected into the patient, the sharpest and farthest extending point (the "needle point"), rather than the part of the point that is closest to the hub (the "needle heel") breaks the patient's skin first. In the case of larger (lower gauge) needles known in the art, the bevel angle is visible to the user's naked eye, and therefore this is fairly easy to do. However, in the case of extremely small needles (for example, higher gauges of about 25 and higher), such as those used for multiple injections as discussed herein, the bevel tip may be very small and difficult or impossible to see.

Thus, a need exists for improved syringes that have detachable and interchangeable needles, such that a used needle may be easily removed from a syringe and replaced with a fresh needle; and that also minimize dead space after injection to preserve as much fluid as possible; as well as kits comprising such syringes and needles that permit easy detachment and interchanging of such parts for users of multiple needles.

### SUMMARY OF THE INVENTION

In certain embodiments, the present invention is directed to a syringe assembly comprising:
(a) a cylindrical barrel having an open proximal end and a distal end, the cylindrical barrel defining an interior space, the interior space comprising a distal portion defining a roof, the interior space configured to receive a plunger capable of sliding relative to the cylindrical barrel from the open proximal end of the cylindrical barrel to the roof;
(b) a plunger having a proximal end and a distal end, the proximal end including a plunger handle and the distal end disposed within the interior space of the cylindrical barrel, the plunger configured to slide relative to the interior space of the cylindrical barrel along the longitudinal axis of the cylindrical barrel, the distal end of the plunger capable of touching the roof on the distal portion of the interior space; and
(c) a needle assembly detachably fixed to the distal end of the cylindrical barrel, the needle assembly comprising a hub and a needle fixed on the hub, wherein the needle comprises a hollow lumen therethrough, the hollow lumen communicating with the interior space of the cylindrical barrel; and
(d) a bevel indicator on the needle assembly;
wherein the distal end of the plunger defines a surface that touches the roof of the cylindrical barrel when the plunger is fully plunged into the cylindrical barrel.

In certain embodiments, the present invention is directed to a syringe assembly comprising:
(a) a cylindrical barrel having an open proximal end and a distal end, the cylindrical barrel defining an interior space, the interior space comprising a distal portion defining a roof, the interior space configured to receive a plunger capable of sliding relative to the cylindrical barrel from the open proximal end of the cylindrical barrel to the roof, the interior space containing fluid;
(b) a plunger having a proximal end and a distal end, the proximal end including a handle portion and the distal end disposed within the interior space of the cylindrical barrel, the plunger configured to slide relative to the interior space of the cylindrical barrel along the longitudinal axis of the cylindrical barrel, the distal end of the plunger having a shape that corresponds to an opposite and complementary shape on the roof of the distal portion of the interior space, such that when the plunger is fully deployed within the cylindrical barrel, the distal end of the plunger and the roof of the distal portion of the interior space mate at an interface to form a seal that forces substantially all of the fluid away from the interface; and
(c) a needle assembly detachably fixed to the distal end of the cylindrical barrel, the needle assembly comprising a hub and a needle fixed on the hub, wherein the needle comprises a hollow lumen therethrough, the hollow lumen communicating with the interior space of the cylindrical barrel; and
(d) a bevel indicator on the needle assembly;
wherein the distal end of the plunger defines a surface that touches the roof of the cylindrical barrel when the plunger is fully plunged into the cylindrical barrel; and wherein the cylindrical barrel and the needle assembly are detachably fixed to each other with a mechanism chosen from: a threading connection, a snap connection and a binary connection.

In certain embodiments, the present invention is directed to a method of delivering a medical or cosmetic fluid to a patient, the method comprising the steps of:
(a) obtaining a syringe assembly comprising a cylindrical barrel configured to receive a slidable plunger therein and a first needle assembly detachably fixed to the cylindrical barrel, the first needle assembly comprising a first needle fixed to a first hub;
(b) inserting the syringe assembly into a container of medical or cosmetic fluid and withdrawing the slidable plunger to load the syringe assembly with a desired amount of the medical or cosmetic fluid;
(c) withdrawing the syringe assembly from the container of drug;
(d) removing the first needle assembly from the cylindrical barrel by separating the first hub from the cylindrical barrel; and
(e) attaching a second needle assembly to the cylindrical barrel, the second needle assembly comprising a second needle fixed to a second hub.

### BRIEF DESCRIPTION OF THE DRAWINGS

**Fig. 1** shows a syringe assembly in accordance with an embodiment of the present invention, wherein the plunger is in a position either during or immediately following the "loading" step and having fluid in its barrel.
**Fig. 2** shows a syringe assembly in accordance with an embodiment of the present invention, wherein the plunger is in a position immediately following deployment - that is, the fluid in the barrel has been fully injected into the body of the patient.
**Fig. 3** shows a side view of a needle assembly in accordance with an embodiment of the present invention.
**Fig. 4** shows a side view of a needle assembly in accordance with another embodiment of the present invention.
**Figs. 5a** and **5b** show a top outside view and a side outside view, respectively, of a needle assembly (including a needle hub) in accordance with a certain embodiment of the invention.
**Fig. 6** shows a side view and a top view of bevel tips of various needles in accordance with certain embodiments of the invention.
**Fig. 7** shows a view of a portion of a distal end of a syringe barrel in accordance with one embodiment of the present invention, showing a portion to which a needle assembly may be detachably fixed.
**Fig. 8** shows a needle assembly of the present invention, which may be detachably fixed to the portion of the barrel shown in **Fig. 7** in a manner that substantially eliminates the dead space.
**Figs. 9a-9c** show views of the distal end of a barrel in accordance with one embodiment of the present invention.
**Figs. 10a** and **10b** show views of the a needle assembly that fits within the distal end of the barrel shown in **Figs. 9a**-**9c**.
**Figs. 11a** and **11b** show views of the distal end of a barrel in accordance with one embodiment of the present invention. **Figs. 11c** and **11d** show views of the a needle assembly that fits within the distal end of the barrel shown in **Figs. 11a** and **11b****.**
**Figs. 12a** and **12b** show views of the distal end of a barrel in accordance with one embodiment of the present invention. **Fig. 12c** shows a view a of the a needle assembly that fits within the distal end of the barrel shown in **Figs. 12a** and **12b****.**
**Figs. 13a** and **13b** show a side view and a cross sectional view, respectively, of a needle assembly in accordance with one embodiment of the present invention. **Figs. 13c** and **13d** show an isometric view and a cross sectional view, respectively, of a distal end of a syringe barrel and a threaded port in accordance with one embodiment of the present invention.
**Figs. 14a** and **14b** show views of the distal end of a barrel in accordance with one embodiment of the present invention.
**Figs. 15a**-**15c** shows views of the a needle assembly that fits within the distal end of the barrel shown in **Figs. 14a** and **14b****.**
**Figs. 16a** and **16b** show views of another syringe assembly in accordance with one embodiment of the present invention.
**Figs. 17a- 17c** show internal and external cross sectional views, respectively, of another syringe assembly in accordance with another embodiment of the present invention.

### DETAILED DESCRIPTION OF THE INVENTION

The syringe assemblies of the present invention are particularly useful for the injection of medical or cosmetic fluid to treat conditions such as, but not limited to, allergies, hypohydrosis, muscle twitches, crossed eyes, cerebral palsy or the like, that require multiple injections along multiple sites on a patient's face or body in a single procedure or series of procedures. As used herein, the terms "medical or cosmetic fluid" refer to any fluid material that is desired to be delivered to a patient's body by injection using a syringe assembly, for the purposes of treating a medical condition or providing a cosmetic benefit. In certain embodiments, the medical or cosmetic fluid may comprise, but is not limited to any of the following: allergenic and allergy-triggering compositions such as dander, or cosmetic compositions such as botulism toxin (in the case of cosmetic fluids such as Botox®) and muscle relaxants and the like.

In certain embodiments, the present invention is directed to a syringe assembly comprising a detachable needle assembly. In certain embodiments, the needle assembly comprises a hub that is fixed to a needle. The hub and needle may be permanently fixed to each other in any of various ways known in the art, including, for example, a jointing medium such as an adhesive.

In certain embodiments, the needle assembly is detachably fixed to the syringe assembly in various ways, as will be described in greater detail. Further, desirable shield apparatuses for storing such assemblies are discussed in great detail in copending U.S. Application No.____________, filed on the same date as the present application and entitled "Shield Apparatuses and Methods for Storing Syringe Assemblies and Needle Assemblies" the disclosure of which is hereby incorporated by reference in its entirety. Further, kits comprising such assemblies are discussed in great detail in copending U.S. Application No. ____________________, filed on the same date as the present application and entitled, "Kits Comprising Syringe Assemblies" the disclosure of which is hereby incorporated by reference in its entirety.

### Low Dead Space

The issue of dead space is an ongoing concern in the field of syringes and other surgical instruments that deliver fluids. In many syringes currently used, the internal stopper or plunger has a conical shape that does not fully contact the internal portion of the tube of the syringe. As a result, a primary concern is that whenever currently used syringes are fully deployed, liquid remains in the syringe, generally in the distal end of the barrel that may comprise a conical or cross-sectionally trapezoidal shape, as well as in the length of the needle itself. This liquid is lost after deployment of the syringe assembly, as there is no way to extract it from the syringe or force it from the barrel through the needle.

Attempts have been made to both minimize dead space and provide detachable needle assemblies. See, for example, U.S. Patent No. 5,782,803 to Jentzen and U.S. Patent Publication No. 2008/0033347 to D'Arrigo. However, these and other references in the art address syringes more traditionally used that have larger needles (generally gauges of far lower than 29) with a capacity of 3 mL and larger. The interchangeability and dead space of lower gauge needles has not been adequately addressed by prior attempts. As discussed herein, higher gauge needles (for example, needles with a gauge of higher than 25, 25-34 or 29, 30, 31, 32 or 33 and even smaller) present unique challenges in the art, including but not limited to their fragility and difficulty to detach due to their small size.

As discussed herein, minimization of dead space is a desirable goal of syringe assemblies, and it has been found here that it can be accomplished via design of the plunger and the syringe barrel. Specifically, the interior of the barrel is configured to receive a plunger, and the plunger has a distal end (which is inserted into the barrel) and a proximal end, which in certain embodiments, comprises a plunger handle to facilitate the user's sliding the plunger along the longitudinal axis of the barrel. The cylindrical barrel itself may further comprise projections on which a user puts his or her hand to facilitate the user's sliding the plunger. The cylindrical barrel comprises an interior cavity, and at the distal portion of the cavity is an interior distal surface, which the plunger touches when fully deployed - that is, fully slid into the barrel such that it can slide no farther in. This interior distal surface of the barrel that the distal end of the plunger ultimately touches when fully deployed, and which represents the farthest surface to which the plunger can travel into the barrel, is often referred to in the art (and will be referred to in the present disclosure) as the "roof" of the barrel.

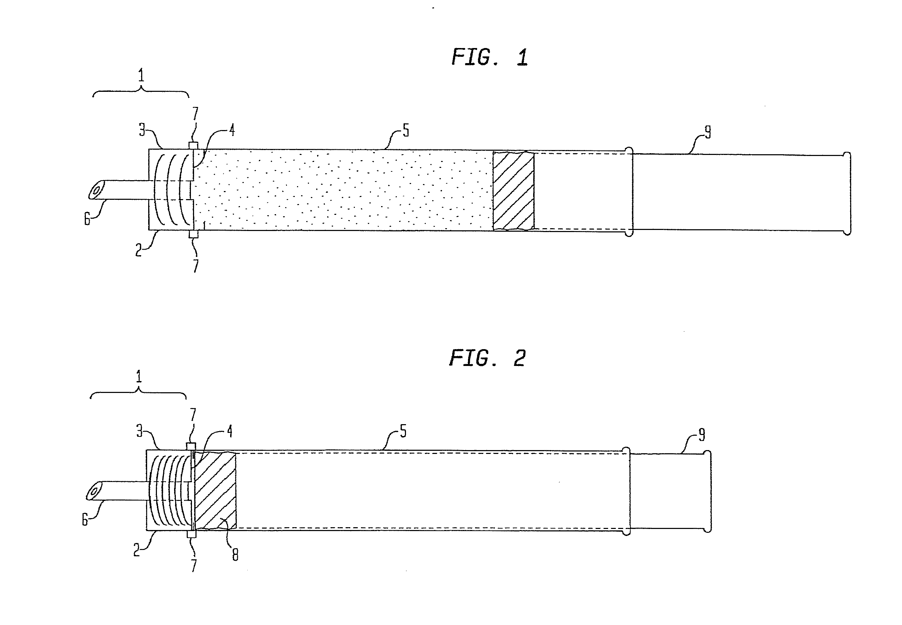

While at least a portion of the roof of the barrel is a closed surface in order to receive the plunger, the roof of the barrel will, in certain embodiments, comprise at least one opening that permits fluid communication of the interior of the barrel with the needle assembly, thus permitting injection of the fluid from the barrel into the body of a patient via the hollow lumen of the needle when the user depresses the plunger incrementally toward the position of being fully deployed, as shown in Fig. 2. The greater the surface area of contact between the distal end of the plunger (when fully deployed) and the roof of the barrel, the less dead space results. In certain embodiments of the present invention, the distal end of the plunger and the roof both have shapes that correspond to and complement each other - that is, the distal end of the plunger has a shape that corresponds to an opposite and complementary shape on the roof of the distal portion of the interior space, such that when the plunger is fully deployed within the cylindrical barrel, the distal end of the plunger and the roof of the distal portion of the interior space mate at an interface to form a seal that forces substantially all of the fluid away from the interface and through the opening through which the interior of the barrel communicates with the needle assembly. The tighter this seal, the more dead space can be minimized and the more fluid can be forced out of the syringe barrel. This corresponding relationship between the distal end of the plunger and the roof is important to ensure that when the plunger is fully deployed into the cylindrical barrel, the plunger and the roof are in substantially full contact with each other.

In certain embodiments, the distal end of the plunger may be one of any other shapes that minimize dead space - for example, partially or substantially conical, trapezoidal, circular, angular, or any shape that is useful for coaxing the fluid down through the barrel and into the needle assembly. For example, the distal end of the plunger may be substantially flat and at a substantially perpendicular angle to the longitudinal axis of the syringe barrel; the corresponding roof of the barrel may also be substantially flat in the same way, such that when the two meet, an optimal amount of fluid may be forced out of the barrel and through the needle.

Further, the distal end of the plunger may constitute a "male" portion that mates with a "female" portion having a complementary surface thereto. In other embodiments, the relative portions can be switched, for example, the plunger may have a "female" portion that mates with a "male" portion of the roof. In still other embodiments, both relative portions may both have one or more of either "male" or "female" portions.

In certain embodiments, the syringe assemblies of the present invention are configured such that less than about 5% of the fluid originally loaded into the barrel is lost to dead space. In various embodiments, this value is less than about 4%, less than about 3%, less than about 2%, or less than about 1%. In certain embodiments, the amount of fluid lost is less than about 0.5 mL, less than about 0.4 mL, less than about 0.1 mL, less than about 0.05 mL or less than about 0.01 mL.

### Detachable Needle Assembly

In various embodiments of the present invention, the syringe assembly comprises a detachable needle assembly. As used herein, "detachable" means that a user can both attach the needle assembly to, and remove the needle assembly from, the distal end of the barrel without damaging either the needle assembly or the barrel or the connection between them.

Thus, in certain embodiments, the syringe assemblies of the present invention will comprise two major components: first, a cylindrical barrel having disposed therein a plunger; and second, a needle assembly comprising a hub fixed to a needle. In certain embodiments, the needle assembly may be detachably fixed to the cylindrical barrel via any of the following mechanisms:
(a) a threaded portion on the distal end of the barrel that mates with a threaded portion of the syringe assembly ("Threading Connection");
(b) a portion on the distal end of the barrel that fits with a corresponding and complementary portion on the syringe assembly by a snapping together ("Snap Connection"); or
(c) male and female portions that are capable of being engaged together and connected by twisting ("Binary Connection").

These mechanisms will be discussed in greater detail herein.

### A. Threading Connection

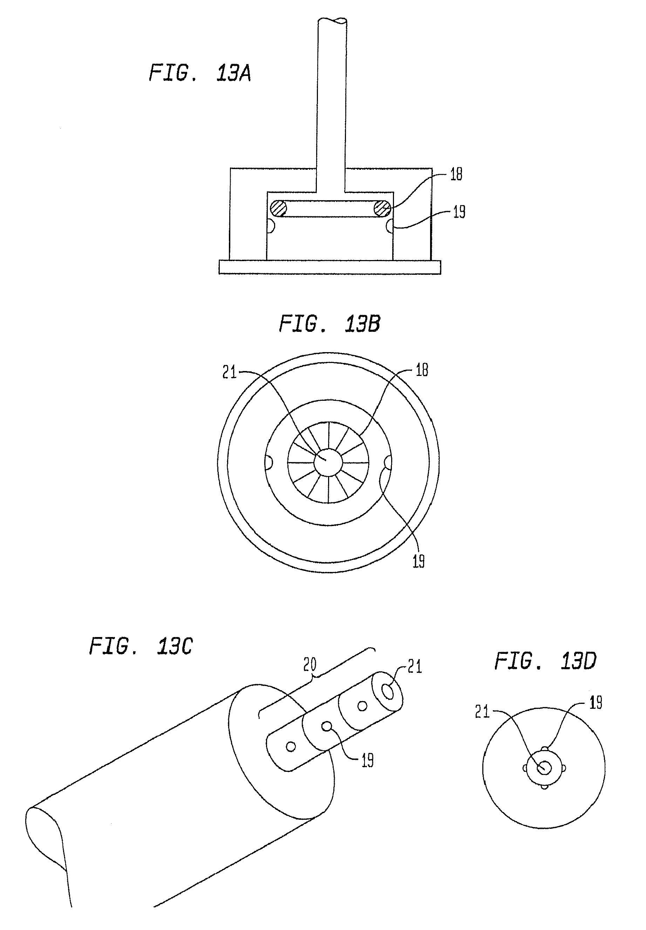

In certain embodiments, the connection between the syringe and the needle may be made by one or more threads on the needle assembly, where the threads correspond to and mate with corresponding threads on the distal end of the syringe assembly at the point of attachment, as with a screw. For example, the distal end of the syringe assembly may terminate in a threaded post (as shown, for example, in **Fig. 13c** as element 20), which fits into the interior of the hub on the needle assembly and which includes an opening 21 that communicates with both the interior of the barrel and, when fully screwed in, with the hollow lumen of the needle on the needle assembly. The reverse may be true in that the needle itself may comprise a threaded port, and the syringe barrel may comprise a hollow tube or port having one or more threads disposed in its interior for mating with the syringe.

As seen in **Figs. 13a****-d,** the threaded portion of either the distal end of the barrel or the needle assembly may further comprise, in certain embodiments, one or more lock washers, O-rings 18, or one or more détentes 19, which can be either concave or protruding convex features. In **Fig. 13a****,** the O-ring can be seen in cross section. **Fig. 13b** provides a top view of the O-ring, showing that it encircles the opening 21. In **Fig. 13c** the detents are convex protrusions on the threaded port 20. They can be seen in cross section in **Fig. 13d****.** These may be useful in ensuring a tight fit when the two portions are screwed together - for example, in certain embodiments, a user may thread the barrel to the needle assembly, and once the two are tightly threaded together, the one or more détentes will "click" to indicate that they have been engaged. Thus, the détentes, when engaged, provide a tactile sensation to the user that the threads have been sufficiently tightened to each other. The presence of the one or more détentes will ensure a tight fit and prevent the two parts from inadvertently unengaging during use of the syringe assembly. The threading is detachable in the sense that a user can easily unthread the two portions when finished, but they will not inadvertently unthread before necessary. In certain embodiments, the threaded portions of the barrel and the needle assembly can be easily threaded together by a user using only one hand.

In certain embodiments, the threading mechanisms connecting the needles to the syringes may further comprise one or more attachments that serve the purpose of locking the two together after threading, such that movement or pushing of the syringe or needle does not cause the needle to "walk off" the syringe during use. Such a locking mechanism may be in the form of a snap, a hook, a clamp flange or other similar mechanism, as well as a temporary and easily removable adhesive or a magnetic attachment.

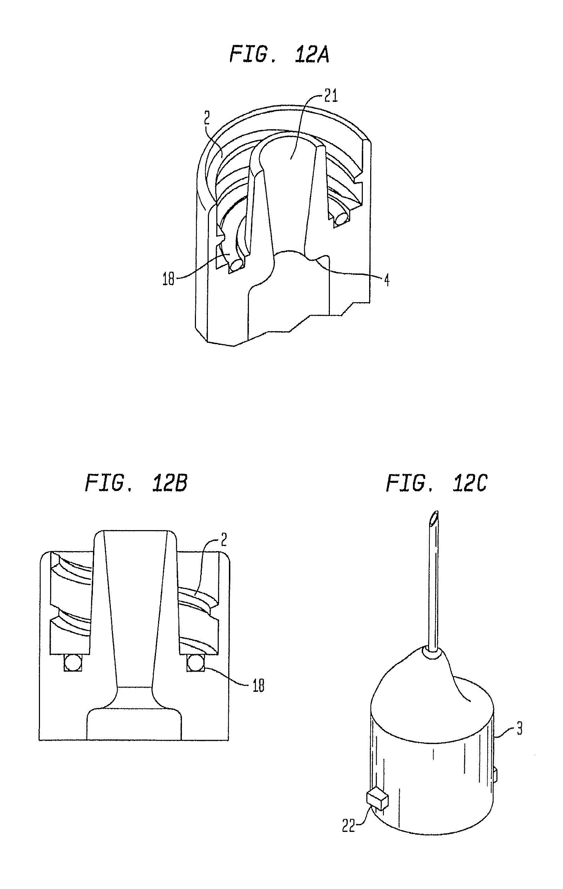

Other examples of threading connections can be seen in **Figs. 12a** and **12b****.** The threads 2 can be on the inside of the distal end of the syringe, as depicted in those two Figures.

**Fig. 12c** shows a needle assembly that would be compatible with the distal end of the syringe as shown in **Figs. 12a** and **12b****.** As can be seen, the needle assembly of **Fig. 12c** includes one or more thread engaging portions that engage the threads 2 of the corresponding distal end of the syringe barrel, and when fully threaded together will be in communication with the roof 4.

### B. "Snap" Connection

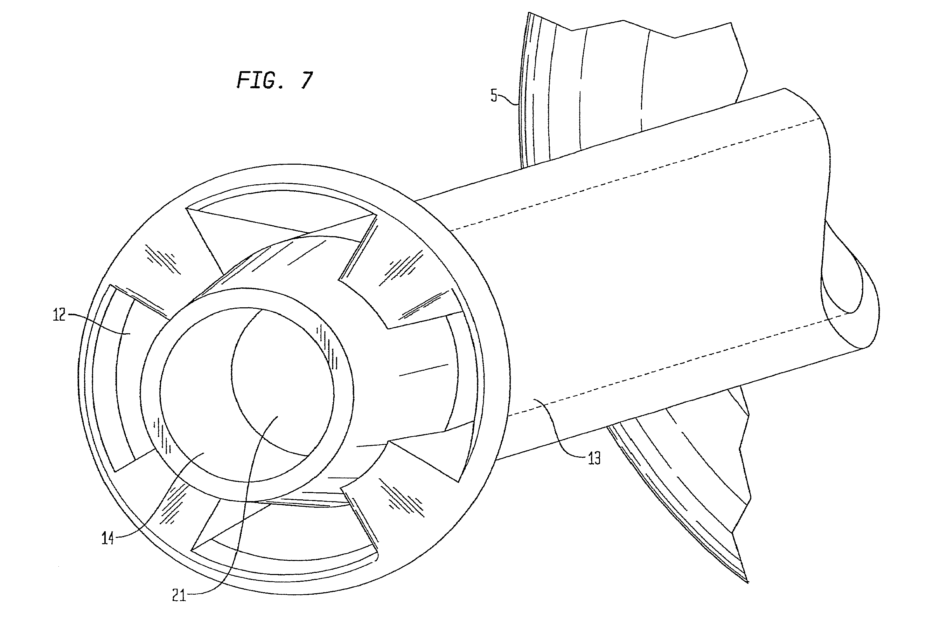

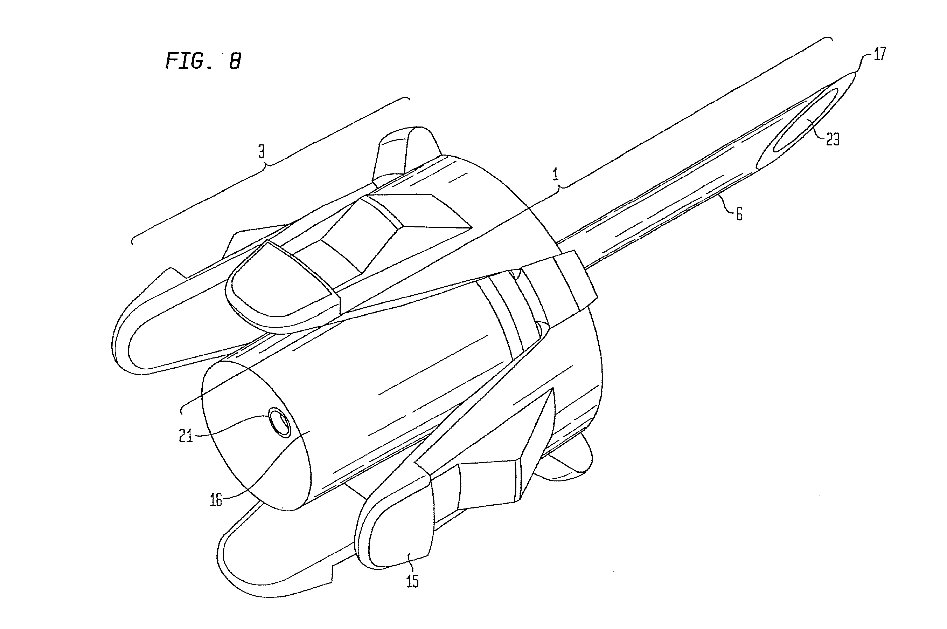

In certain embodiments, the connection between the syringe assembly and the barrel may be made with corresponding mating parts on the needle (or attachment thereto) and the syringe, for example, "male" and "female" parts that mate together by a "click" or "snap" mechanism. In such embodiments, either of the needle assembly or the distal end of the barrel may be configured to have a slightly larger diameter than the other, such that one of the two can fit over the other and, in certain embodiments, deform and then pop into place in a manner that the two are not easily separated during use of the syringe assembly. In certain embodiments, be locked or joined tightly into place. Such a mechanism is shown, for example, in **Figs 7** and **8****.** **Fig. 7** shows a connection on the distal end of a cylindrical barrel 5. In the embodiment pictured here, there is a cylindrical port 13 that extends slightly beyond the roof of the barrel, and that provides a mating surface for the needle assembly to join to, and on which the hub of the needle assembly will eventually rest. Here, a female portion 14 on the port 13 defines an opening 21 which is in communication with the roof of the barrel (not pictured) and after attachment with the needle assembly, will also be in communication with the hollow lumen of the needle. The port 13 also includes snap enclosures 12 around its perimeter, which have slightly flexible ends (that is, ends that give slightly when pulled in a horizontal direction, but preferably pop back into place once placed around an elongated item such as a syringe barrel) to engage corresponding edges on the end of the syringe and lock the needle assembly into place for stability during use. In this case there are four snap enclosures 12, but all embodiments of the present invention are not so limited. As can be seen in **Fig. 8****,** a needle assembly 1 comprising a hub 3 can be snapped onto the port 13 of **Fig. 7****,** in accordance with the present invention. The needle assembly includes tabs 15 around its perimeter. The number of tabs may be equal to the number of snap enclosures 12, such that a user can push the needle assembly onto the port 13 by mating the male portion 16 of the needle assembly 1 with the female portion 14 of **Fig. 7****.** The tabs 15 are engaged in the snap enclosures 12 to provide a tight fit, therefore contacting the opening on the distal end of the barrel with the opening 21 on the interior of the needle assembly, which leads to the hollow lumen inside the needle 23. However, the needle assembly can be easily detached from the port 13 by squeezing the tabs so as to disengage the tabs from the snap enclosures.

### C. Binary Connection

In various embodiments, the connection is of a binary nature - that is, the corresponding ends of the syringe or needle (or attachment thereto) have locking portions that can be mated by inserting one portion into a corresponding and complementary portion of another and then making a "twist" to lock the two in place. Generally, the "twist" action may be less than a full 360 degree twist - that is, a fraction of the outer circumference of the locking portions. In various embodiments, the "twist" action can be accomplished by twisting to an amount of about 45 to about 100 degrees, about 50 to about 95 degrees or about 90 degrees. By "binary" it is meant that the corresponding parts can be configured in two possible ways - locked and unlocked or fixed and unfixed or attached and unattached.

A kit according to these embodiments of the invention may comprise, as stated above, a configuration wherein a user can easily detach and interchange needles by inserted the end of a used needle into a chamber, giving the syringe a fractional turn (in various embodiments, a quarter turn, a half turn or a turn of about 45 degrees, about 90 degrees, about 180 degrees or about 270 degrees), thereby detaching the used needle. The user can then insert the syringe into a different chamber, give a similar turn and engage a fresh needle that is ready for use once it is pulled out of the chamber.

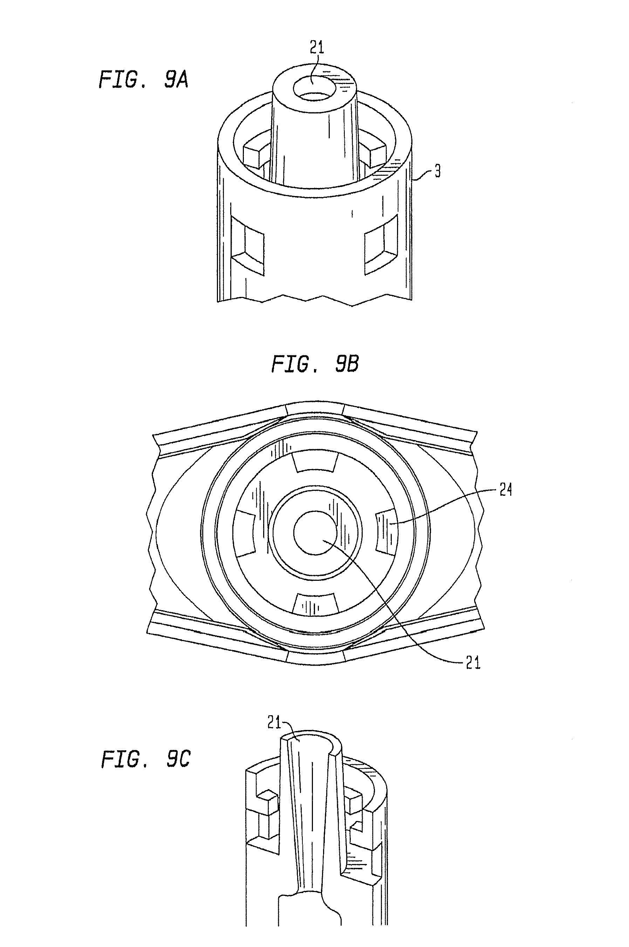

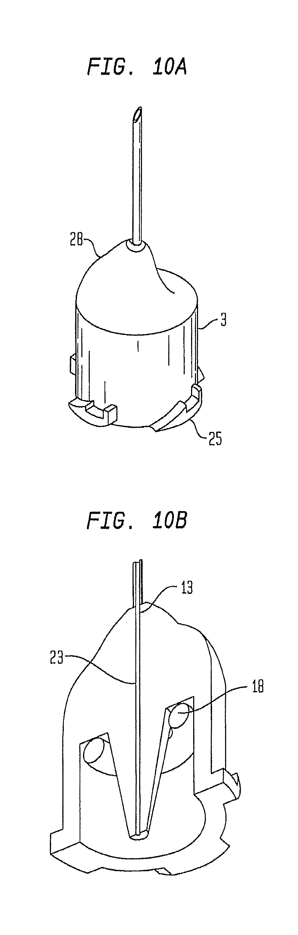

In another embodiment, the port or hub may be configured such that the needle assembly can be attached to the distal end of the cylindrical barrel by a turn - for example, a quarter turn - to tighten the seal between the two. This is shown, for example, in **Figs. 9a****-c** and **Figs. 10a****-b,** as well as **Figs. 16a** and **16b****.** The distal end of the barrel is shown in various views in **Figs. 9a****-c.** In the embodiments pictured therein, both the distal end of the barrel and the needle hub have a male and female protrusion that, when engaged together, eliminate the dead space in the barrel. The distal end of the barrel has protrusions 24 along its inner perimeter (in this case, four of them but the embodiments of the present invention are not so limited). As can be seen in **Fig. 10a****,** the protrusions correspond with, and are complementary with, engaging protrusions 25 on the hub of the needle assembly. The tab and notch protrusion allow for a quick lock by twisting the needle hub into the syringe tip housing, for example, in a clockwise direction, while pushing down. The tab rides up the incline of the protrusion 25. As can be seen from **Fig. 10b****,** an O-ring 18 is present on the interior of the hub of the needle assembly, and the hollow lumen 13 of the needle is in direct communication with the opening 21 in the distal end of the barrel. The O-ring can both prevent leaking and provide a spring resistance to hold the tab down within the notch, locking them in place. To release, the user can push the hub down against the O-ring's spring resistance, thereby releasing the tab from the notch, and then twist the hub (for example, in a counterclockwise direction) and pulling the hub off. In this embodiment,

In certain embodiments, the hub of the needle assembly has a bulge 28 at the tip to create a snap tip with a needle cap. The clip is strong enough to secure the needle while handling, but weak enough to release the needle hub when engaged with the rest of the syringe assembly.

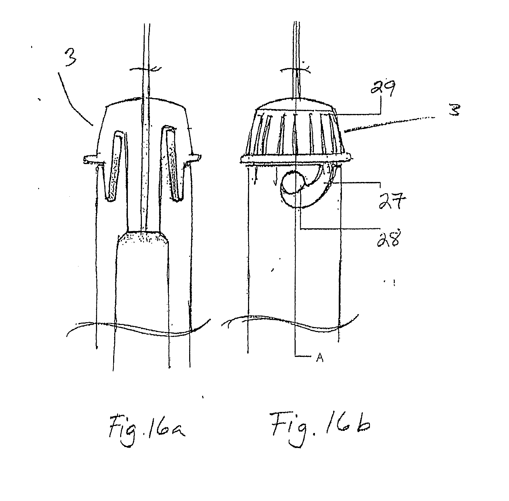

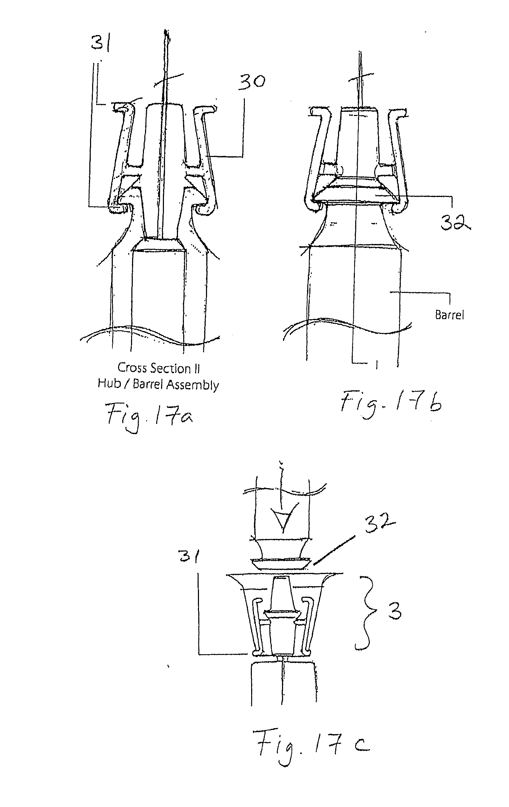

In **Figs. 16a** and **16b****,** a hub 3 comprises vertical ribs 29. A track 27, into which a pin 28 is inserted, and then engaged with a partial turn of the hub. In **Figs. 17a- 17c****,** one or more toggles 30 is present Each toggle has one or more toggle lips 31, which catch the needle within the hub and hold it in place. In the embodiments shown therein, the hub 3 comprises a barrel snap ring 32

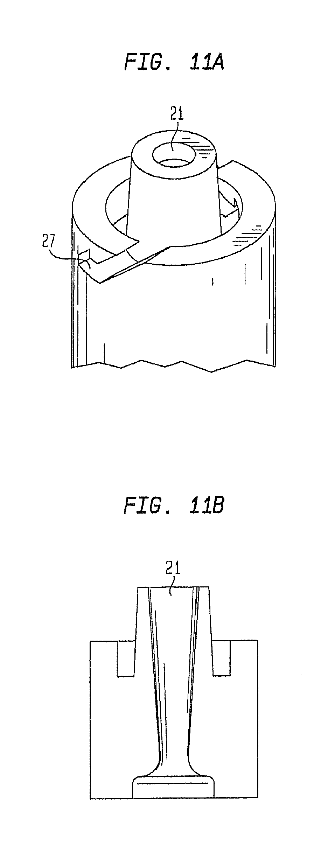

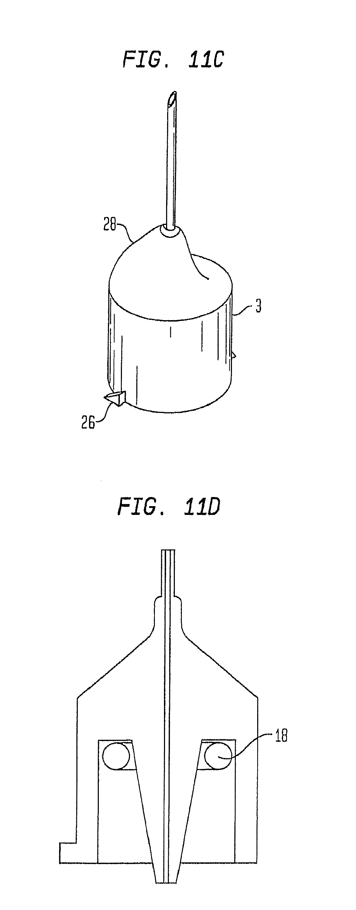

Other exemplary embodiments of such configurations can be seen in **Figs. 11a** and 11b, which show external and internal views, respectively, of the distal end of a syringe barrel as well as **Figs. 11c** and **11d****,** which show the corresponding syringe assemblies that fit therein. As can be seen from **Fig. 11c****,** the syringe assembly's exterior comprises one or more engaging fins 26, which fit into one or more engaging fin enclosures or tracks 27 disposed on the distal end of the syringe barrel, as shown in **Fig. 11a****.** This "mating" is followed by the user's performing a twist of the two parts relative to each other, to slide the engaging fin 26 into the engaging fin enclosure, locking the syringe assembly to the distal end of the syringe barrel. To disengage the two, the user can simply grasp one of the syringe assembly or syringe barrel in each hand, push the two toward each other to separate the engaging fin 26 from the fin enclosure or track 27, and twist in the opposite direction, separating the two parts. As shown in **Fig. 11d****,** an O-ring may be present in the interior of the hub of the syringe assembly.

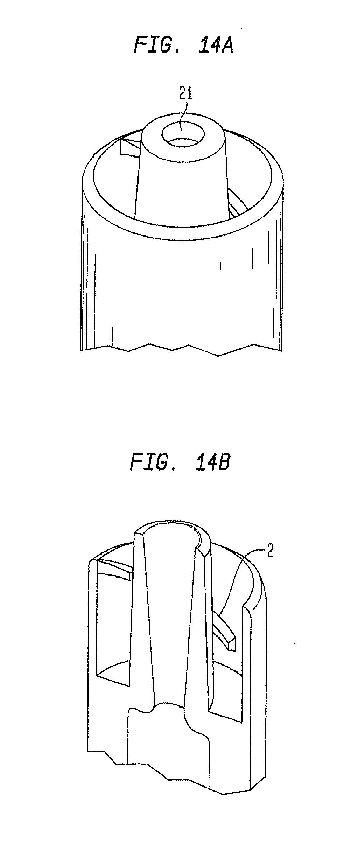

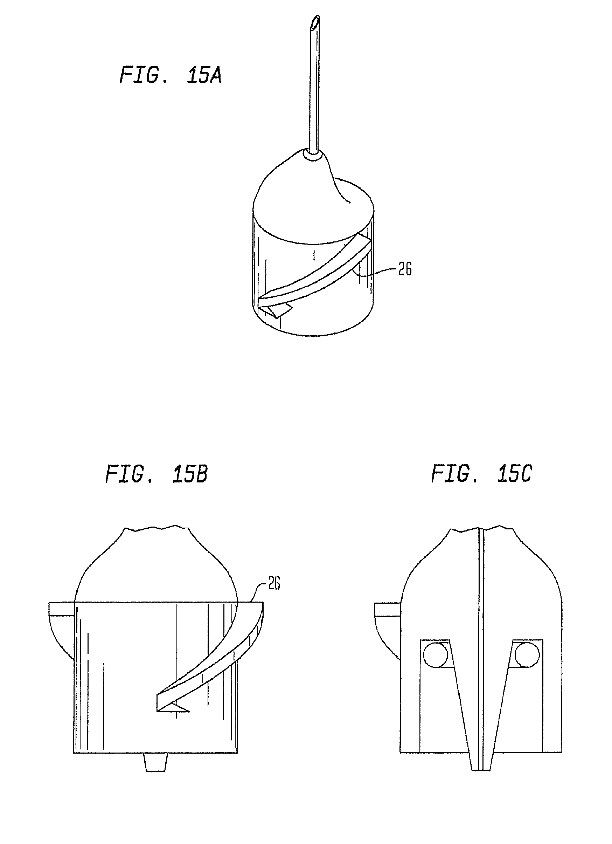

Another example of a syringe assembly of the present invention can be seen in **Figs. 14a** and **14b****,** as well as **Figs. 15a-15c****.** In this assembly, which is essentially a hybrid between a threading connection and a binary connection. In this embodiment, one or more engaging fins 26 are present on the surface of the hub of the needle assembly, such that the total amount the user must "twist" the needle assembly amounts to less than 360 degrees. The corresponding threads 2 are corresponding and complementary to the engaging fins 26. Thus, this is a threading connection in that there are threads, but it is also a binary connection in that the threads do not require complete rotation (or "screwing") of the two parts together.

Thus, in various embodiments, the syringe assemblies of the present invention are configured to optimize each of the three factors discussed above - that is: (1) the surface of the distal end of the piston is in substantially full contact with the roof of the barrel's interior surface when fully inserted therein to minimize dead space; (2) the detachable needle assembly detaches from the cylindrical barrel at a point on the distal end of the cylindrical barrel to allow interchangeability of needle assemblies; and (3) a bevel indicator is located on the needle assembly so as to assist the user in injecting the needle in a way that best serves the needs of the patient. That is, it has been found that the combination of these three factors - low dead space, detachable needle assembly and bevel indicator - is optimal. It is advantageous that the syringes of the present invention are directed to a syringe that has both the efficiency of low dead space coupled with the convenience of a detachable needle and a bevel indicator

**Fig. 1** shows a cross sectional side view of a syringe assembly in accordance with an embodiment of the present invention, wherein the plunger is in a position immediately following the "loading" step - that is, fluid has been fully loaded into the syringe assembly, and it is ready to be injected into a patient. As can be seen in **Fig. 1****,** the needle assembly has been threadably engaged onto the distal end of the cylindrical barrel. The threads 2 are visible here, although in certain embodiments they would not be visible under the exterior surface of the needle hub 3. When fully threadably engaged onto the distal end of the barrel 5, the needle 6 is in fluid communication with the roof 4. In certain embodiments, the user can, after threadably engaging the needle assembly to the cylindrical barrel, trigger an additional locking mechanism 7 to ensure that the engagement is fixed without the fear that the needle assembly will come unthreaded during use. In **Fig. 1****,** the additional locking mechanism 7 is shown as two clamps, each on opposite sides of the hub; however, the locking mechanism are not so limited in all embodiments.

**Fig. 2** shows a syringe assembly in accordance with an embodiment of the present invention, wherein the plunger is in a position immediately following deployment - that is, the fluid in the barrel has been fully injected into the patient's body, and the distal end of the plunger 8 is fully in contact with the roof 4 of the barrel. In the embodiment shown in **Fig. 2****,** the locking mechanisms 7 are not necessary, as the threads 2 are configured such that the user will tighten them until feeling a single "click," which indicates that the détentes have been engaged.

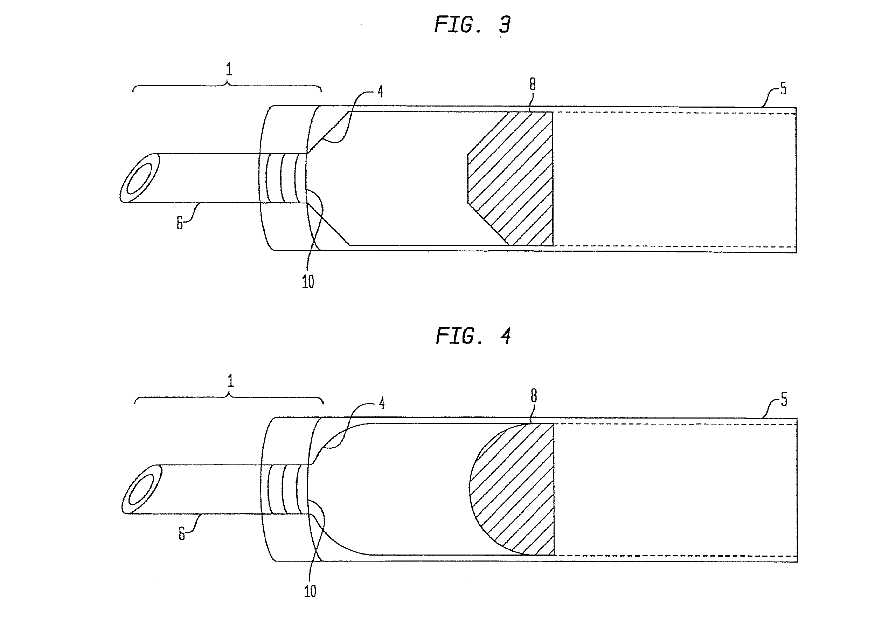

**Fig. 3** shows a side view of a needle assembly in accordance with an embodiment of the present invention. The needle assembly 1 is fully threadably engaged on the distal end of the barrel 5. The distal end of the plunger 8 is configured such that it provides a shape that is complementary and opposite to the shape of the roof 4. In **Figs. 3** and **4****,** the opening 10 through which the needle 6 is in fluid communication with the interior of the barrel can be more clearly seen. **Fig. 4** shows a side view of a needle assembly in accordance with another embodiment of the present invention. The needle assembly 1 is fully threadably engaged on the distal end of the barrel 5. The distal end of the plunger 8 is configured such that it provides a shape that is complementary and opposite to the shape of the roof 4; here, the roof 4 is configured in a semicircular configuration to fit with the distal end of the plunger.

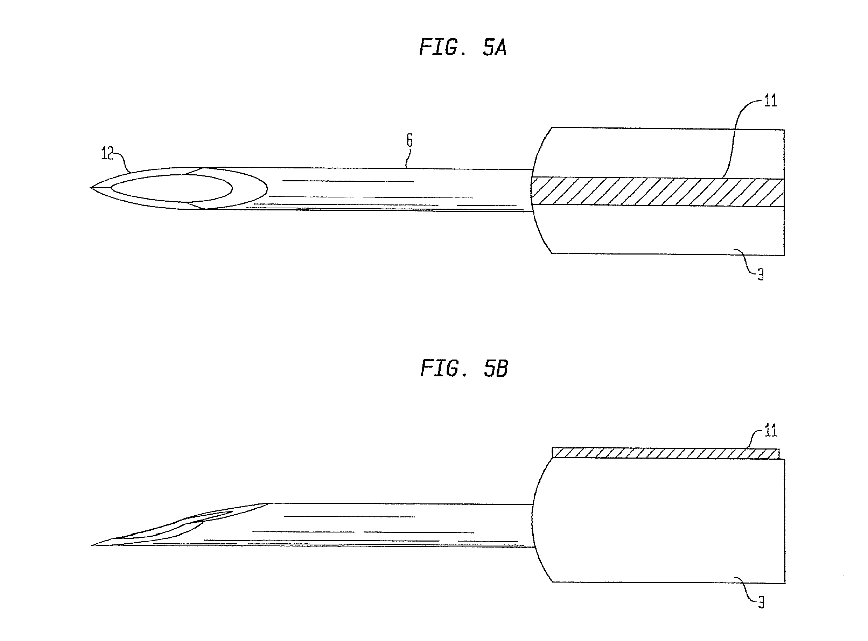

**Fig. 5** shows a side view of another needle assembly in accordance with an embodiment of the present invention. In the embodiment depicted, the bevel indicator 11 is a painted line that is at least in part of a color, texture or pattern that contrasts with that of the surface of the hub, such that a user can align it at substantially the top of the hub when getting ready to inject the needle into a patient. As can be seen from **Fig. 5****,** when the bevel indicator is substantially at the top of the hub as viewed from above by a user, the bevel will be positioned in the optimal configuration for injection into the patient's body with its sharpest point entering first, thus causing minimum discomfort to the patient.

In certain embodiments, the syringe further comprises a gasket in its internal portion. In certain embodiments, an "O-ring" is present. The O-ring provides a spring resistance for locking the needle hub to the distal end of the syringe, and also provides a tight seal that prevents leakage of the liquid contained within the barrel of the syringe. In various embodiments, a coating seal made of a material such as silicone may be used in conjunction with, or instead of, the O-ring.

### Bevel Indicator

As discussed herein, many needles comprise a beveled tip, such that the tip is at an angle. Bevel tips are highly desirable for many injection purposes. In certain embodiments, it is highly desirable to inject the needle with its bevel point entering the patient first, to minimize discomfort. While for larger needles (for example, needles having 18-25 gauge) the user can make this determination with the naked eye, this is almost impossible to do with small needles. Thus, in certain embodiments of the present invention, the needle hub, or any other point along the needle or syringe, may comprise a bevel indicator. The syringe assembly may comprise a bevel indicator that is located at any point along the syringe assembly (including on any portion of the needle assembly). In particular, where the syringe assembly is detachable from the barrel, in certain embodiments the bevel indicator may be present at least in part on the hub, and may be, for example, a line or a dot or other easily discernible indicator, and may be colored in a manner that its contrast with the color of the hub makes it easily visible to a user, and/or raised or recessed or having a texture in a manner that its contrast with the texture of the hub and thus visible or discernible by touch to the user.

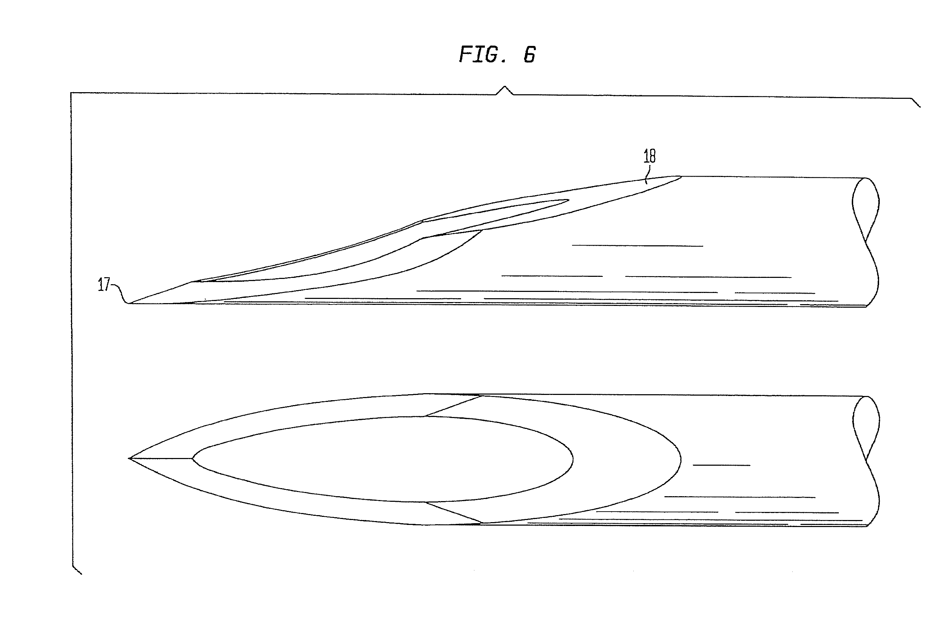

In this manner, the user can use the bevel indicator to determine the best way to inject the needle. For an example, Fig. 6 illustrates this concept. Generally, a user will prefer that the needle first enter the patient's body at the needle point 17, rather than the needle heel 18. As can be seen, failing to allow the needle point 17 to enter first may lead to a jagged entry point for the needle and unnecessary pain and/or injury to the patient. As can be seen from Fig. 5, if the user lines up the needle assembly such that the bevel indicator 11 on the hub 3 is substantially on the top of the hub, this will help ensure that the needle point enters the patient's body first. Note that in the embodiment pictured in Fig. 5, the bevel indicator is a line, but the present invention is not so limited. In this manner, the user can use the bevel indicator to minimize the patient's discomfort.

### Ease of Use

When undergoing procedures such as Botox® injections, allergy injections or insulin injections quickly, or a patient may be suffering pain or discomfort due to muscle spasms or a related medical condition necessitating a series of injections rapidly administered (for example, a series of injections to treat allergies or cerebral palsy). In such situations, a patient may be impatient, agitated or emotional (this is especially true if the patient is a child, elderly or otherwise having diminished capacity), and anything that will streamline the process of injecting, reloading and re-injecting is likely to be desirable from the standpoint of patient comfort. In particular, embodiments of the present invention provide an easy way for a user (such as the medical or spa staff, or the patient himself or herself) to go through the entire process of unwrapping a syringe from its packaging, loading the drug (for embodiments wherein the drug is not pre-loaded into the syringe), preparing the appropriate needle for injection, injecting the drug, detaching and discarding the used needle and re-loading a fresh needle as necessary, all with minimal effort.

In various embodiments, a user may load the syringe assembly with a larger (lower gauge) needle, and then detach the larger needle assembly from the syringe barrel, then attach a smaller (higher gauge) needle assembly. Lower gauge (larger) needles are more desirable for loading, as they can load more fluid in a faster amount of time, with less danger of breaking or dulling the needle. In various embodiments, the first (loading) needle may be a lower gauge (larger) needle; for example, 25 gauge or lower, 18 to 25 gauge, or 18, 20 or 22 gauge. In various embodiments, the second (injecting) needle may be a higher gauge (smaller) needle; for example, higher than 25 gauge, 26-34 gauge, or 29, 30, 31, 32 or 33 gauge.

In various embodiments, the syringes of the present invention may include a plunger handle or a syringe flange, both of which may be configured for ease of use - for example, a plunger handle or syringe flange having a loop that can easily be grasped using a single hand, or comprising a soft or pliant material, such as an elastomeric material, 1 that provides a comfortable grip for the user, such as one that yields to the pressure of a user's hand when gripped.

The syringe barrels in the syringe assemblies of the present invention may have fluid volume capacities, in various embodiments, of about 2 to about 5 mL, about 3 mL, about 2 mL, about 1 mL, about 0.5 mL or about 0.3 mL. It has been found that the assemblies of the present invention provide unexpected benefits when used with very small volume barrels and very small (higher gauge) needles.

The syringe assemblies of the present invention may be made of any materials that are useful for medical devices, including those that are inert, stable, disposable and/or can easily be sterilized. Preferably they will not cause undue discomfort or allergic reactions in users or patients. Examples of useful materials are glass, polymeric materials such as plastic (including but not limited to materials comprising polypropylene, polyethylene, polystyrene, polyethylene terephthalate, or low density or high density forms of any of the foregoing), natural or synthetic rubbers, elastomeric materials, fiberglass, glass, metal and the like.

All embodiments described herein are illustrative and in no way limit the scope of the invention, and the invention may be embodied in other forms not explicitly described here, without departing from the spirit thereof.

### ADDITIONAL DESCRIPTION

1. A syringe assembly comprising:
   (a) a cylindrical barrel having an open proximal end and a distal end, the cylindrical barrel defining an interior space, the interior space comprising a distal portion defining a roof, the interior space configured to receive a plunger capable of sliding relative to the cylindrical barrel from the open proximal end of the cylindrical barrel to the roof;
   (b) a plunger having a proximal end and a distal end, the proximal end including a plunger handle and the distal end disposed within the interior space of the cylindrical barrel, the plunger configured to slide relative to the interior space of the cylindrical barrel along the longitudinal axis of the cylindrical barrel, the distal end of the plunger capable of touching the roof on the distal portion of the interior space; and
   (c) a needle assembly detachably fixed to the distal end of the cylindrical barrel, the needle assembly comprising a hub and a needle fixed on the hub, wherein the needle comprises a hollow lumen therethrough, the hollow lumen communicating with the interior space of the cylindrical barrel; and
   (d) a bevel indicator on the needle assembly;
      wherein the distal end of the plunger defines a surface that touches the roof of the cylindrical barrel when the plunger is fully plunged into the cylindrical barrel.
2. The syringe assembly of clause 1, wherein the bevel indicator is located on the hub of the needle assembly.
3. The syringe assembly of clause 2, wherein the bevel indicator comprises a marking on the hub of the needle assembly that indicates the relative position of the needle point.
4. The syringe assembly of clause 1, comprising medical or cosmetic fluid.
5. The syringe assembly of clause 1, wherein the medical or cosmetic fluid comprises botulism toxin.
6. The syringe assembly of clause 1, wherein the volume capacity of the syringe assembly is less than about 3 mL.
7. The syringe assembly of clause 1, wherein the volume capacity of the barrel is less than about 2.75 mL.
8. They syringe assembly of clause 1, wherein the needle has a gauge of higher than 25.
9. The syringe assembly of clause 1, wherein the distal end of the barrel comprises a threaded portion, and the interior of the hub of the needle assembly comprises a complementary threaded portion that mates with the threaded portion of the distal end of the barrel.
10. The syringe assembly of clause 1, wherein one or more of the threaded portions comprises a detente.
11. The syringe assembly of clause 1, wherein the distal end of the barrel comprises a male portion and the hub of needle assembly comprises a female portion, and wherein the male portion and female portion snap together to connect the distal end of the barrel with the needle assembly.
12. A syringe assembly comprising:
   (a) a cylindrical barrel having an open proximal end and a distal end, the cylindrical barrel defining an interior space, the interior space comprising a distal portion defining a roof, the interior space configured to receive a plunger capable of sliding relative to the cylindrical barrel from the open proximal end of the cylindrical barrel to the roof, the interior space containing fluid;
   (b) a plunger having a proximal end and a distal end, the proximal end including a handle portion and the distal end disposed within the interior space of the cylindrical barrel, the plunger configured to slide relative to the interior space of the cylindrical barrel along the longitudinal axis of the cylindrical barrel, the distal end of the plunger having a shape that corresponds to an opposite and complementary shape on the roof of the distal portion of the interior space, such that when the plunger is fully deployed within the cylindrical barrel, the distal end of the plunger and the roof of the distal portion of the interior space mate at an interface to form a seal that forces substantially all of the fluid away from the interface; and
   (c) a needle assembly detachably fixed to the distal end of the cylindrical barrel, the needle assembly comprising a hub and a needle fixed on the hub, wherein the needle comprises a hollow lumen therethrough, the hollow lumen communicating with the interior space of the cylindrical barrel; and
   (d) a bevel indicator on the needle assembly;
   wherein the distal end of the plunger defines a surface that touches the roof of the cylindrical barrel when the plunger is fully plunged into the cylindrical barrel; and wherein the cylindrical barrel and the needle assembly are detachably fixed to each other with a mechanism chosen from: a threading connection, a snap connection and a binary connection.
13. A method of delivering a medical or cosmetic fluid to a patient, the method comprising the steps of:
   (a) obtaining a syringe assembly comprising a cylindrical barrel configured to receive a slidable plunger therein and a first needle assembly detachably fixed to the cylindrical barrel, the first needle assembly comprising a first needle fixed to a first hub;
   (b) inserting the syringe assembly into a container of medical or cosmetic fluid and withdrawing the slidable plunger to load the syringe assembly with a desired amount of the medical or cosmetic fluid;
   (c) withdrawing the syringe assembly from the container of drug;
   (d) removing the first needle assembly from the cylindrical barrel by separating the first hub from the cylindrical barrel; and
   (e) attaching a second needle assembly to the cylindrical barrel, the second needle assembly comprising a second needle fixed to a second hub.
14. The method of clause 13, comprising the additional step of:
   (f) injecting the syringe assembly into the patient.
15. The method of clause 13, wherein the first needle has a gauge of 25 or lower.
16. The method of clause 15, wherein the first needle has a gauge of 18, 20, 22 or 25.
17. The method of clause 13, wherein the second needle has a gauge of higher than 25.
18. The method of clause 17, wherein the second needle has a gauge of 25-34.

## Claims

1. A syringe assembly comprising:
(a) a cylindrical barrel having an open proximal end and a distal end, the cylindrical barrel defining an interior space, the interior space comprising a distal portion defining a roof, the interior space configured to receive a plunger capable of sliding relative to the cylindrical barrel from the open proximal end of the cylindrical barrel to the roof;
(b) a plunger having a proximal end and a distal end, the proximal end including a plunger handle and the distal end disposed within the interior space of the cylindrical barrel, the plunger configured to slide relative to the interior space of the cylindrical barrel along the longitudinal axis of the cylindrical barrel, the distal end of the plunger capable of touching the roof on the distal portion of the interior space;
**characterized in that** the volume capacity of the syringe assembly is less than about 3 mL, and the distal end of the plunger having a shape that corresponds to an opposite and complementary shape on the roof of the distal portion of the interior space such that when the plunger is fully deployed within the cylindrical barrel, the percentage of fluid lost to dead space is less than 5%, the syringe assembly further comprising:
(c) a needle assembly detachably fixed to the distal end of the cylindrical barrel, the needle assembly comprising a hub and a needle fixed on the hub, wherein the needle has a gauge of higher than 25 and comprises a hollow lumen therethrough, the hollow lumen communicating with the interior space of the cylindrical barrel; and
(d) a bevel indicator on the needle assembly.

2. The syringe assembly of claim 1, wherein when the plunger is fully deployed within the cylindrical barrel, the percentage of fluid lost to dead space is less than 1%.

3. The syringe assembly of claim 1, wherein when the plunger is fully deployed within the cylindrical barrel, the amount of fluid lost to dead space is less than 0.01mL.

4. The syringe assembly of any of claims 1-3, wherein the distal end of the plunger has a trapezoidal cross-sectional shape that corresponds to an opposite and complementary shape on the roof of the distal portion of the interior space.

5. The syringe assembly of any of claims 1-3, wherein the distal end of the plunger has a semi-circular cross-sectional shape that corresponds to an opposite and complementary shape on the roof of the distal portion of the interior space.

6. The syringe assembly of any of claims 1-5, wherein the distal end of the barrel comprises a threaded portion, and the interior of the hub of the needle assembly comprises a complementary threaded portion that mates with the threaded portion of the distal end of the barrel, wherein one or more of the threaded portions comprises a detent.

7. The syringe assembly of any of claims 1-5, wherein the distal end of the barrel comprises a male portion and the hub of needle assembly comprises a female portion, and wherein the male portion and female portion snap together to connect the distal end of the barrel with the needle assembly.

8. The syringe assembly of claim 7, wherein the male portion comprises snap enclosures and the female portion comprises tabs, the tabs being engaged in the snap enclosures and being squeezable to disengage the tabs from the snap enclosures.

9. The syringe assembly of any of claims 1-5, wherein the hub of the needle assembly comprises tab and notch protrusions and the distal end of the barrel comprises protrusions along an inner perimeter, wherein the barrel protrusions ride up an incline of the tab and notch protrusions and lock by twisting, and wherein the hub of the needle assembly releases by a push to release the barrel tabs from the notch of the tab and notch protrusions.

10. The syringe assembly of any of claims 1-5, wherein the hub of the needle comprises a bulge configured to snap together with a needle cap.

11. The syringe assembly of any of claims 1-5, wherein the exterior of the needle assembly comprises engaging fins and the distal end of the cylindrical barrel comprises engaging fin enclosures, the engaging fins sliding into the engaging fin enclosures and locking by a twist relative to each other of less than 360 degrees.

12. The syringe assembly of any of claims 1-5, wherein the hub of the needle assembly comprises vertical ribs, the hub engaged with a partial turn of a pin inserted into a track.

13. The syringe assembly of any of claims 1-5, wherein the distal end of the barrel comprises a barrel snap ring and the hub comprises toggles configured to engage the barrel snap ring.

14. The syringe assembly of any of claims 1-5, further comprising a mechanism that locks the cylindrical barrel and the needle assembly together, the mechanism being a magnetic locking mechanism.
